# EUROPÄISCHE PATENTSCHRIFT

(11) **EP 3 111 892 B1**
(45) Veröffentlichungstag und Bekanntmachung des Hinweises auf die Patenterteilung: **12.09.2018**
(21) Anmeldenummer: 15174449.7
(22) Anmeldetag: 30.06.2015
(51) Int. Cl.: A61F 2/34, A61F 2/30

(54) **IMPLANTAT UND VERFAHREN ZUM BESCHICHTEN EINES IMPLANTATGRUNDKÖRPERS**
IMPLANT AND METHOD FOR COATING AN IMPLANT BASE BODY
IMPLANT ET PROCÉDÉ DE REVÊTEMENT D'UN CORPS DE BASE D'IMPLANT

(43) Veröffentlichungstag der Anmeldung: 04.01.2017
(73) Patentinhaber: Jossi Holding AG, 8546 Islikon (CH)
(72) Erfinder: Meyenhofer, Andreas, 8255 Schlattingen (CH); Gugler, Christian, 8500 Frauenfeld (CH); Nadler, Daniel, 8442 Hettlingen (CH)
(74) Vertreter: Hepp Wenger Ryffel AG

(56) Entgegenhaltungen:
- WO-A2-2006/094270
- DE-A1- 4 211 343
- DE-A1- 19 755 536
- GB-A- 2 288 537
- US-A- 4 854 496
- US-A- 4 883 491

## Beschreibung

Die vorliegende Erfindung betrifft ein Implantat zur Implantation in einen Knochen sowie Verfahren zum Beschichten eines Implantatgrundkörpers gemäss den Oberbegriffen der unabhängigen Ansprüche.

Zur Verankerung von Implantaten, insbesondere von Endoprothesen in Knochen, ist eine Vielzahl von Oberflächenstrukturen bekannt. Diese reichen von aufgerauten Oberflächen über spezielle Beschichtungen bis hin zur Ausstattung der Implantatoberfläche mit komplexen Strukturelementen.

So ist es beispielsweise bei Hüftgelenksprothesen von Vorteil, wenn Implantatteile, insbesondere die im Hüftknochen angebrachte Gelenkpfanne, ausreiss- und drehfest im Knochengewebe verankert sind. Während einige Gelenkpfannen mit Schrauben fixiert werden, erfolgt bei anderen die Befestigung mittels Knochenzement, während weitere durch Einschlagen oder Einschrauben direkt im Knochen gehalten werden.

Um das Ausreissverhalten sowie die Drehfestigkeit einer mittels Knochenzement, Einschrauben oder Einschlagen befestigten Gelenkpfanne zu erhöhen, ist die Oberfläche einer solchen üblicherweise mit einer rauen Oberflächenstruktur versehen. So können durch zumeist spanabhebende Verfahren Strukturelemente in die Oberfläche eingearbeitet werden, die eine gleichmässige Verteilung der auftretenden Kräfte, sowohl beim Einsetzen als auch bei späterer Belastung der Gelenkpfanne, ermöglichen, und so ein Drehen oder gar ein Ausreissen verhindern. Im Falle eines Einschlagens des Implantates ermöglichen Strukturelemente darüber hinaus ein Einwachsen des Knochens in deren Zwischenräume.

So ist durch die WO 2012/126944 A1 beispielsweise ein Hüftgelenkpfannenimplantat in Form einer Kugelkalottenschale bekannt, dessen Oberflächenstruktur eine Vielzahl von Strukturelementen umfasst, die durch jeweils mehrere, sich überkreuzende Rillen mit entgegengesetzter Steigung gebildet sind.

Die US 5,645,593 (= GB 2 288 537 A) beschreibt ebenfalls ein Knochenimplantat mit Strukturelementen. Allerdings sind die Zwischenräume zwischen diesen Strukturelementen vollständig mit einem Granulat gefüllt. Das Granulat bildet eine poröse Beschichtung, welche eine gute Sekundärfixierung durch Osteointegrätion ermöglicht. Durch das Vorhandensein von Strukturelementen wird dabei ein Abscheren der porösen Beschichtung beim Einsetzen des Implantates in den Knochen verhindert. Da die Vertiefungen zwischen den Strukturelementen allerdings vollständig mit porösem Material gefüllt sind, resultiert eine reduzierte Primärfixation des Implantates und eine erhöhte Belastung des Knochens beim Einsetzen des Implantates.

Die US 4,883,491 zeigt ebenfalls eine künstliche Hüftgelenkpfanne, die allerdings mit Gewindezügen zum Einschrauben in einen Knochen versehen ist. Die besagten Gewindezüge sind durch Bereiche mit einer porösen Beschichtung unterbrochen. Allerdings ist die poröse Beschichtung nicht gegen ein Abscheren im Zuge des Einschraubens des Implantates geschützt. Die poröse Beschichtung kann daher beim Transport des Implantates oder bei dessen Handhabung im Operationssaal abgerieben werden. Die dadurch freigesetzten Partikel können im Körper des Patienten Schädigungen verursachen und im schlimmsten Fall in die Artikulationsflächen der Gelenkprothese gelangen, wo sie dessen Verschleiss signifikant erhöhen. Dies kann bis zum Versagen des gesamten Gelenkimplantates führen.

Die US 4,854,496 beschreibt ein mit einem porösen Metall beschichtetes Implantat und ein Verfahren zu dessen Herstellung. Dabei wird eine poröse Beschichtung als vorgefertigte Einlage in einen Implantatgrundkörper eingesetzt.

Die DE 197 55 536 A1 beschreibt eine Hüftgelenkspfanne. Dabei wird vorgeschlagen, dass die Lagerschale der Hüftgelenkspfanne in dem Bereich ihrer Oberfläche, mit dem sie in den Hüftknochen eingesetzt wird, mit einer Beschichtung aus einem biokompatiblen Metall oder einer biokompatiblen Metalllegierung überzogen ist.

Die WO 2006/094270 A2 beschreibt ein Verfahren zur Herstellung einer Hüftgelenksschale. Dabei wird eine Arbeitsfläche bereitgestellt, die eine Hüftgelenksschale definiert. Danach wird eine Materialschicht in einer ersten Zusammensetzung auf die Arbeitsfläche aufgesprüht und anschliessend wird die Hüftgelenksschale von der Arbeitsfläche abgenommen.

Es ist die Aufgabe der Erfindung, die Nachteile im Stand der Technik zu überwinden.

Insbesondere ist es eine Aufgabe der Erfindung, ein verbessertes Implantat zur Implantation in einen Knochen zu schaffen, welches eine Oberflächenstruktur mit einer porösen Beschichtung aufweist. Dabei soll das Implantat eine verbesserte Primärfixation in einem Knochen ermöglichen und gleichzeitig eine Oberfläche mit verbessertem Abriebverhalten aufweisen.

Diese Aufgaben werden durch ein Implantat gelöst, welches die Merkmale im Anspruch 1 aufweist, sowie durch ein Verfahren zum Beschichten eines Implantatgrundkörpers gemäss den Ansprüchen 9 und 11.

Die Erfindung betrifft ein Implantat, insbesondere ein Hüftgelenkpfannenimplantat, zur Implantation in einen Knochen. Das Implantat weist mindestens teilweise eine Oberflächenstruktur auf, welche Erhebungen und Vertiefungen umfasst. Die Vertiefungen weisen Zonen mit einer porösen Beschichtung auf.

Die Erfindung zeichnet sich dadurch aus, dass
a) die Erhebungen unbeschichtete Zonen aufweisen, die an die beschichteten Zonen in den Vertiefungen angrenzen,
b) oder dass die Erhebungen beschichtete Zonen aufweisen, die an die beschichteten Zonen in den Vertiefungen angrenzen, wobei die Schichtdicke der porösen Beschichtung auf den Erhebungen kleiner ist als in den Vertiefungen,
c) oder dass die Erhebungen beschichtete Zonen aufweisen, die an die beschichteten Zonen in den Vertiefungen angrenzen, wobei die Beschichtung auf den Erhebungen weniger porös ist als in den Vertiefungen,
d) oder dass die Erhebungen beschichtete Zonen aufweisen, die an die beschichteten Zonen in den Vertiefungen angrenzen, wobei die Abriebfestigkeit der porösen Beschichtung auf den Erhebungen grösser ist als in den Vertiefungen.

Dabei erstreckt sich die poröse Beschichtung der Kontur der Vertiefungen folgend.

Die Begriffe "Erhebung" und "Vertiefung" sind dabei im relativen Bezug zueinander zu verstehen. Der Begriff "Abriebfestigkeit" bezeichnet in diesem Zusammenhang die Widerstandsfähigkeit der porösen Beschichtung gegenüber mechanischer Beanspruchung, insbesondere Reibung. Sie wird hauptsächlich durch deren Rauheit und Härte, aber auch von der Haftfestigkeit am Implantatgrundkörper, bestimmt. Experimentell kann die Abriebfestigkeit durch standardisierte Verfahren wie z.B. durch abrasive Methoden oder Abreisstests bestimmt werden.

Die poröse Beschichtung muss nicht zwingend über die gesamte Schichtdicke porös sein. Denkbar ist auch eine stellenweise Kompaktierung, so dass eine geschlossene Oberfläche entsteht. Da sich beschichtete Zonen mit geringer Abriebfestigkeit oder verhältnismässig grosser Schichtdicke, wenn überhaupt, nur im Bereich der Vertiefungen der Oberflächenstruktur erstrecken, und damit geschützt sind, ist das gesamthafte Abriebverhalten der porösen Beschichtung, beispielsweise beim Einsetzen des Implantates in einen Knochen, erheblich verbessert. Da die Vertiefungen allerdings nicht vollständig mit porösem Material gefüllt sind, ermöglicht das Implantat dennoch eine gute Primärverankerung im Knochen. Die poröse Beschichtung folgt aus diesem Grund stets der Kontur der Vertiefungen, was nicht zwingend heisst, dass die Beschichtung ein exaktes Abbild der Vertiefungen sein muss. Der wesentliche Aspekt besteht darin, dass unabhängig von der Dicke der porösen Beschichtung stets eine Aussenkontur mit Vertiefungen und Erhöhungen verbleibt.

Beim Implantat kann die Schichtdicke der porösen Beschichtung zwischen 15 % und 50 % der Profiltiefe betragen. Wenn das Verhältnis zwischen Profiltiefe und Schichtdicke in diesem Bereich liegt, resultiert ein besonders vorteilhaftes Zusammenspiel zwischen Primärfixation und Sekundärfixation. Unter dem Begriff "Profiltiefe" wird im vorliegenden Zusammenhang der mittlere Versatz zwischen höchsten Punkten der einzelnen Erhebungen und den tiefsten Punkten der einzelnen Vertiefungen verstanden.

In den Vertiefungen kann die Schichtdicke der porösen Beschichtung von 0.1 mm bis 0.5 mm betragen. Eine derartige Schichtdicke ermöglicht eine gute Sekundärfixierung im Knochen, wobei die mechanische Stabilität des Implantates gewährleistet bleibt.

Das Implantat kann eine Grundstruktur bestehend aus Titan, Zirkonium, Niob, Tantal oder Legierungen davon, Kobalt-Chrom-legierung, Medizinstahl, Keramik oder Polyethylen umfassen. Auch die poröse Beschichtung kann mit Ausnahme von Medizinalstahl, Keramik und Polyethylen aus diesen Materialien bestehen. Derartige Materialien sind bei der Herstellung von Knochenimplantaten weit verbreitet. Sie lassen sich mit gängigen Methoden auf dem Gebiet der Herstellung gattungmässiger Implantate bearbeiten und weisen eine hervorragende Verträglichkeit beim Patienten auf.

Darüber hinaus können derartige Materialien eine Stützstruktur für die nachwachsenden Knochenzellen bilden, wodurch das natürliche Knochenwachstum begünstigt wird. Durch die Verwendung derartiger Materialien kann entsprechend der Heilungsprozess des Knochens nach einer Implantation sowie die Bildung einer stabilen Verbindung zwischen dem Implantat und dem Knochen unterstützt werden. In besonders bevorzugten Fällen können die genannten Materialien sogar einen direkten, funktionellen, strukturellen Verbund mit dem lebenden Knochengewebe eingehen. Dadurch wird eine äusserst feste Verankerung des Implantates in einem Knochen erzielt.

Die poröse Beschichtung kann eine Partikelbeschichtung sein und vorzugweise Partikel aufweisen, die sphärisch, kubisch oder zylindrisch in der Form sind. Partikelbeschichtungen weisen eine vorteilhafte Porosität auf. Darüber hinaus sind im Stand der Technik zuverlässige Methoden zum Aufbringen von Partikelbeschichtungen auf Implantatgrundkörper bekannt, wie beispielsweise das atmosphärische Plasmaspritzverfahren (APS) oder das Vakuum Plasmaspritzverfahren (VPS). Durch die Wahl der Partikelform lässt sich die Implantatoberfläche auf den jeweiligen Anwendungsbereich abstimmen.

Die poröse Beschichtung kann, insbesondere grobe und feine, Partikel mit einer mittleren Grösse von 200 µm bis 500 µm aufweisen. Eine derartige Partikelgrösse ergibt eine Porosität, die besonders gut für ein Einwachsen des Knochens geeignet ist. Durch die Wahl der Partikelgrösse lässt sich die Implantatoberfläche auf eine jeweilige Anwendung abstimmen.

Die Porosität der Beschichtung kann derart eingestellt sein, dass sie am Grund der Vertiefungen grösser ist als im Bereich der Seitenflanken der Erhebungen oder auf den Erhebungen. Durch diese Verteilung der Porosität wird ein Abrieb der Partikel beim Einsetzen des Implantates in einen Knochen weiter vermieden.

Des Weiteren betrifft die Erfindung ein Verfahren zum Beschichten eines Implantatgrundkörpers, insbesondere zur Herstellung eines oben beschriebenen Implantates, umfassend die Schritte
- Bereitstellen eines unbeschichteten Grundkörpers, wobei der Grundkörper mindestens teilweise eine Oberflächenstruktur aufweist, welche Erhebungen und Vertiefungen umfasst,
- Selektives aufbringen einer porösen Beschichtung, derart, dass beschichtete Zonen im Bereich der Vertiefungen an unbeschichtete Zonen im Bereich der Erhebungen angrenzen, und dass sich die poröse Beschichtung der Kontur der Vertiefungen folgend erstreckt.

Ein derartiges Verfahren hat den Vorteil, dass nach Aufbringen der porösen Beschichtung nicht ein Teil davon wieder abgetragen werden muss.

Das selektive Aufbringen der porösen Beschichtung kann dabei durch Auftragen einer Schicht im Bereich der Erhebungen erfolgen, die ein Anhaften der porösen Beschichtung verhindert. Eine solche Schicht wird auch als Maskierung bezeichnet. Diese Variante stellt eine einfache Methode zum selektiven Aufbringen der porösen Beschichtung im Bereich der Vertiefungen dar, die ohne spezialisierte Werkzeuge auskommt.

Ein selektives Aufbringen der porösen Beschichtung ist allerdings auch durch Einstellen von Parametern des Prozesses denkbar, wie beispielsweise der Strahlrichtung, Fokus oder Intensität eines Partikelstrahls.

Ein alternatives Verfahren zum Beschichten eines Implantatgrundkörpers, insbesondere zur Herstellung eines oben beschriebenen Implantates, umfasst die folgenden Schritte:
- Bereitstellen des unbeschichteten Grundkörpers, wobei der Grundkörper mindestens teilweise eine Oberflächenstruktur aufweist, welche Erhebungen und Vertiefungen umfasst,
- Aufbringen einer porösen Beschichtung im Bereich mit der Oberflächenstruktur,
- Abtragen der porösen Beschichtung im Bereich der Erhebungen, derart dass
   a) die Erhebungen beschichtete Zonen aufweisen, die an die beschichteten Zonen in den Vertiefungen angrenzen, und dass die Schichtdicke der porösen Beschichtung auf den Erhebungen kleiner ist als in den Vertiefungen,
   b) oder dass die Erhebungen beschichtete Zonen aufweisen, die an die beschichteten Zonen in den Vertiefungen angrenzen, und dass die Abriebfestigkeit der porösen Beschichtung auf den Erhebungen grösser ist als in den Vertiefungen,
   und dass sich die poröse Beschichtung der Kontur der Vertiefungen folgend erstreckt.

Ein derartiges Verfahren stellt eine besonders einfache und kostengünstige Methode zur Herstellung eines oben beschriebenen Implantates dar. Dadurch dass in einem Verfahrensschritt eine poröse Beschichtung im Bereich mit der Oberflächenstruktur aufgebracht wird, ohne zwischen den Bereichen der Erhebungen und den Bereichen der Vertiefungen zu unterscheiden, sind beim besagten Verfahren herkömmliche Beschichtungsmethoden, die dem Fachmann bestens bekannt sind, anwendbar.

Das Abtragen der porösen Beschichtung im Bereich der Erhebungen kann dabei durch Kugelstrahlen, Abscheren, Bürsten, Drehen, Fräsen, Schleifen, Gleitschleifen (Trowalisieren), chemisches oder elektrochemisches Abtragen erfolgen. Diese Techniken sind auf dem Gebiet der Herstellung gattungmässiger Implantate weit verbreitet und gebräuchlich.

Das Aufbringen der porösen Beschichtung kann durch ein Plasmabeschichten, insbesondere durch ein atmosphärisches Plasmaspritzverfahren (APS) oder ein Vakuum Plasmaspritzverfahren (VPS), durch Präzipitation, oder Additive Manufacturing erfolgen. Diese Methoden sind auf dem Gebiet weit verbreitet und erlauben je nach den zu erfüllenden Erfordernissen ein zuverlässiges, wahlweise selektives, auf jeden Fall jedoch effizientes Auftragen der porösen Beschichtung.

Die oben genannten Verfahren können einen zusätzlichen Schritt, welcher das Sintern des mit der porösen Beschichtung beschichteten Grundkörpers beinhaltet, umfassen. Dadurch wird einerseits eine hohe Festigkeit der porösen Beschichtung an sich, andererseits aber auch eine gute Anhaftung am Implantatgrundkörper, erreicht.

Weitere Vorteile und Einzelmerkmale der Erfindung ergeben sich aus der nachfolgenden Beschreibung zweier Ausführungsbeispiele und aus den Zeichnungen.

Es zeigen schematisch:
- Figur 1:: Querschnittsprofil der Oberflächenstruktur eines erfindungsgemässen Implantates;
- Figur 2:: Querschnittsprofil der Oberflächenstruktur eines alternativen Ausführungsbeispiels eines erfindungsgemässen Implantates;
- Figur 3:: Unbeschichteter Implantatgrundkörper für ein erfindungsgemässes Implantat;
- Figur 4:: Erfindungsgemässes Implantat aufweisend eine Oberflächenstruktur mit einem Querschnittsprofil gemäss Figur 1;
- Figur 5:: Schnittbild der Oberflächenstruktur eines erfindungsgemässen Implantates;
- Figur 6:: Vergrössertes Schnittbild der Oberflächenstruktur eines erfindungsgemässen Implantates.

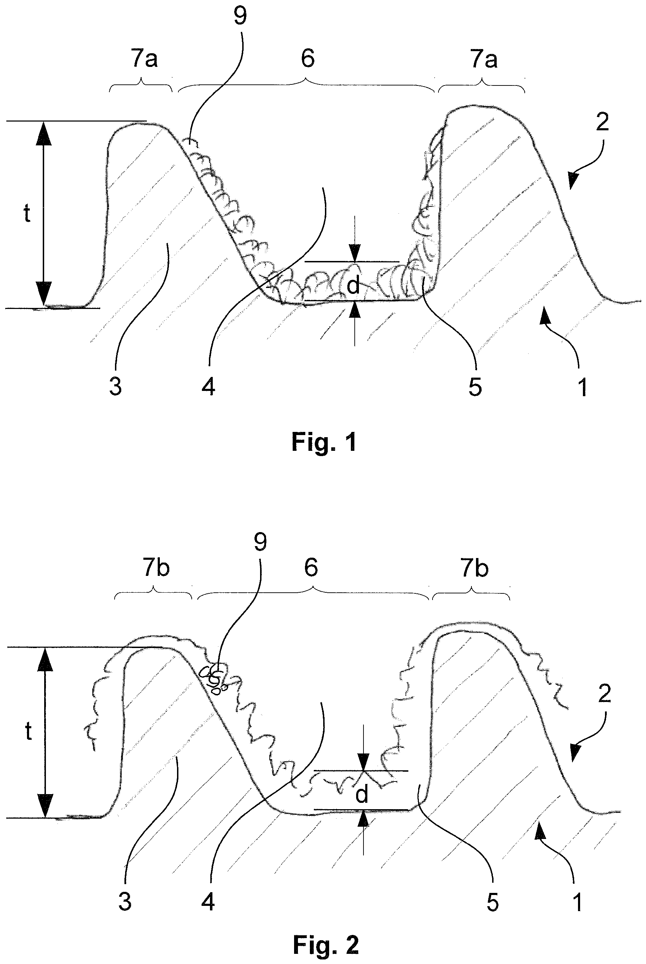

Wie aus Figur 1 hervorgeht, umfasst die Oberflächenstruktur 2 eines erfindungsgemässen Implantates 1 Erhebungen 3 und Vertiefungen 4. Im vorliegenden Fall erstrecken sich die mit einer porösen Beschichtung 5 beschichteten Zonen 6 nur über die Vertiefungen 4, während die Erhebungen 3 unbeschichtete Zonen 7a aufweisen. Die Oberflächenstruktur 2 weist eine Profiltiefe t und die poröse Beschichtung 5 in den beschichteten Zonen 6 eine Schichtdicke d auf.

Wie Figur 2 zu entnehmen ist, unterscheidet sich das dort dargestellte Ausführungsbeispiel dahingehend von demjenigen aus Figur 1, dass sowohl die Erhebungen 3 als auch die Vertiefungen 4 mit einer porösen Beschichtung 5 beschichtet sind. Allerdings weisen die Erhebungen 3 beschichtete Zonen 7b auf, in denen die Schichtdicke der porösen Beschichtung 5 kleiner ist als in den Vertiefungen 4. Die Beschichtung kann hier aber auch einfach eine geringere Porosität bzw. eine grössere Kompaktheit aufweisen, als in der Vertiefung 4. Darum ist die Abriebfestigkeit der porösen Beschichtung 5 in den Zonen 7b grösser als in den Vertiefungen 4. Bei der dargestellten porösen Beschichtung 5 handelt es sich um eine Partikelbeschichtung, die sich aus den Partikeln 9 zusammensetzt.

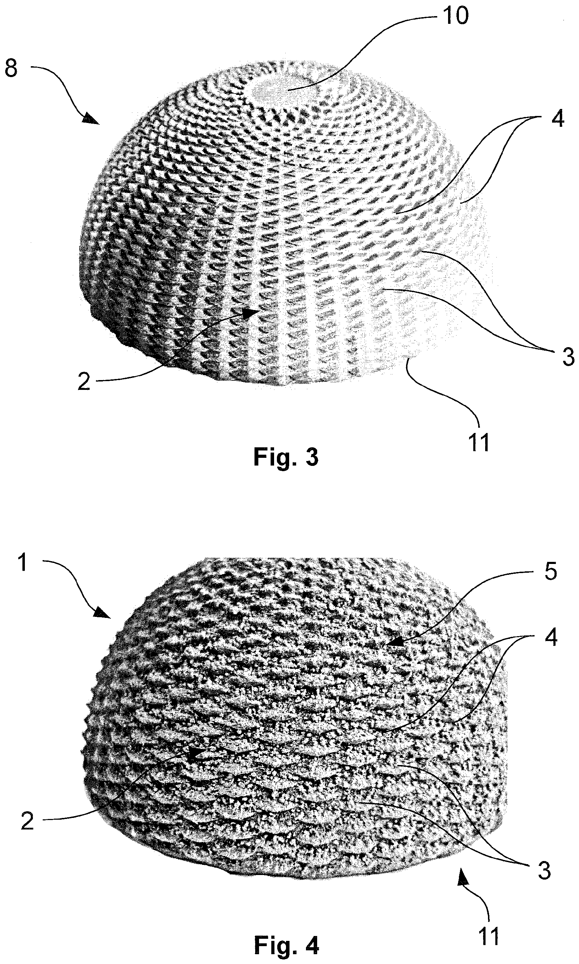

Der Implantatgrundkörper 8 gemäss Figur 3 wurde durch Anbringen von rillenförmigen Vertiefungen 4 auf einem Metallrohling hergestellt. Es handelt sich um einen kugelkalottenschalenförmigen Implantatgrundkörper 8 mit einem Pol 10 und einem Äquator 11. Die Oberflächenstruktur 2 weist neben den bereits angesprochenen Vertiefungen 4 Erhebungen 3 auf.

Das in Figur 4 abgebildete erfindungsgemässe Implantat 1 wurde aus einem Grundkörper 8 von der Art des in Figur 3 abgebildeten hergestellt, wobei ein Verfahren gemäss Anspruch 11 angewendet wurde. Entsprechend wurde nach Bereitstellen eines unbeschichteten Grundkörpers 8 im Bereich mit einer Oberflächenstruktur, welche sich in diesem Fall vom Pol 10 bis zum Äquator 11 erstreckt, eine poröse Beschichtung 5 aufgetragen. Daraufhin wurde die poröse Beschichtung 5 im Bereich der Erhebungen 3 wieder derart abgetragen, dass die Schichtdicke der porösen Beschichtung 5 auf den Erhebungen 3 kleiner ist als in den Vertiefungen 4. Durch das Abtragen werden Partikel mit geringerer Haftung entfernt, was zu einer Verringerung der Schichtdicke führt. Dadurch ist die die Abriebfestigkeit der porösen Beschichtung 5 auf den Erhebungen 3 grösser als in den Vertiefungen 4.

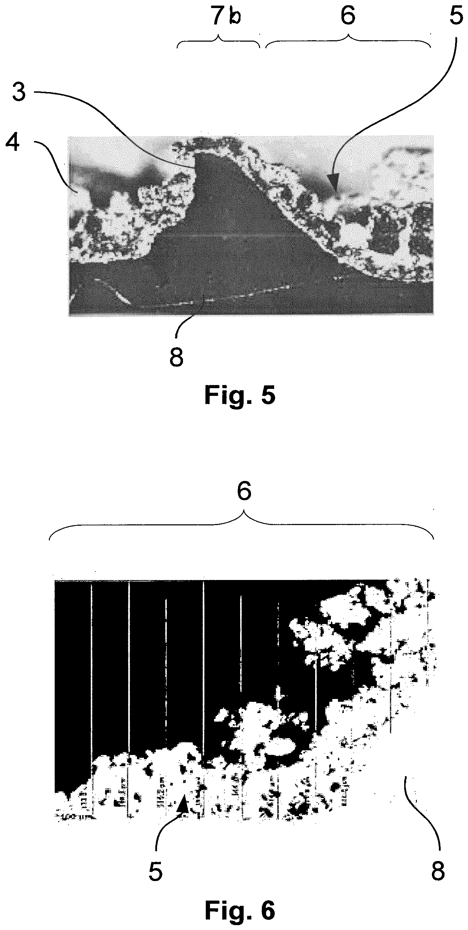

Die Figuren 5 und 6 zeigen Schnittbilder der Oberflächenstruktur erfindungsgemässer Implantate.

Gemäss Figur 5 ist die Erhebung 3 im Querschnitt asymmetrisch ausgebildet. Die Beschichtung 5 bestehend aus Titanpartikeln ist auf der steil abfallenden linken und auf der etwas flacheren rechten Flanke unterschiedlich verteilt. Die dunklen Bereiche der Beschichtung entsprechen Poren zwischen den hellen Titanpartikeln. Deutlich sichtbar ist die Schichtdicke in den Vertiefungen 4 grösser, als auf dem Scheitel der Erhebung 3.

In Figur 6 ist gegenüber Figur 5 noch weiter vergrössert eine offenporige Struktur der Beschichtung 5 sichtbar. Die hellen Bereiche der Titanpartikel bilden eine zerklüftete Struktur, die das Einwachsen von Knochensubstanz ermöglicht.

## Patentansprüche

1. Implantat (1), insbesondere Hüftgelenkpfannenimplantat, zur Implantation in einen Knochen, wobei das Implantat (1) mindestens teilweise eine Oberflächenstruktur (2) aufweist, welche Erhebungen (3) und Vertiefungen (4) umfasst, wobei die Vertiefungen (4) Zonen (6) mit einer porösen Beschichtung (5) aufweisen, wobei
a) die Erhebungen (3) unbeschichtete Zonen (7a) aufweisen, die an die beschichteten Zonen (6) in den Vertiefungen angrenzen,
b) oder die Erhebungen (3) beschichtete Zonen (7b) aufweisen, die an die beschichteten Zonen (6) in den Vertiefungen angrenzen, wobei die Schichtdicke der porösen Beschichtung (5) auf den Erhebungen kleiner ist als in den Vertiefungen (4),
c) oder die Erhebungen beschichtete Zonen (7b) aufweisen, die an die beschichteten Zonen (6) in den Vertiefungen angrenzen, wobei die Beschichtung auf den Erhebungen weniger porös ist als in den Vertiefungen,
d) oder die Erhebungen (3) beschichtete Zonen (7b) aufweisen, die an die beschichteten Zonen (6) in den Vertiefungen angrenzen, wobei die Abriebfestigkeit der porösen Beschichtung (5) auf den Erhebungen grösser ist als in den Vertiefungen (4),
und wobei sich die poröse Beschichtung (5) der Kontur der Vertiefungen (4) folgend erstreckt.

2. Implantat (1) nach Anspruch 1, **dadurch gekennzeichnet, dass** die Schichtdicke (d) der porösen Beschichtung (5) in den Vertiefungen (4) zwischen 15 % und 50 % der Profiltiefe (t) beträgt.

3. Implantat (1) nach einem der Ansprüche 1 oder 2, **dadurch gekennzeichnet, dass** die Schichtdicke (d) der poröser Beschichtung (5) in den Vertiefungen (4) zwischen 0.2 mm und 0.5 mm beträgt.

4. Implantat (1) nach einem der Ansprüche 1 bis 3, **dadurch gekennzeichnet, dass** das Implantat (1) einen Grundkörper (8) bestehend aus Titan, Zirkonium, Niob, Tantal, oder Legierungen davon, Kobalt-Chrom-Legierung, Medizinstahl, Keramik oder Polyethylen umfasst.

5. Implantat (1) nach einem der Ansprüche 1 bis 4, **dadurch gekennzeichnet, dass** das Implantat (1) eine poröse Beschichtung (5) bestehend aus Titan, Zirkonium, Niob, Tantal, oder Legierungen davon, Kobalt-Chrom-Legierung aufweist.

6. Implantat (1) nach einem der Ansprüche 1 bis 5, **dadurch gekennzeichnet, dass** die poröse Beschichtung (5) eine Partikelbeschichtung ist und vorzugsweise Partikel (9) aufweist, die sphärisch, kubisch oder zylindrisch in der Form sind.

7. Implantat (1) nach Anspruch 6, **dadurch gekennzeichnet, dass** die poröse Beschichtung (5) Partikel (9) mit einer mittleren Grösse von 200 µm bis 500 µm aufweist.

8. Implantat (1) nach einem der Ansprüche 1 bis 7, **dadurch gekennzeichnet, dass** die Porosität der Beschichtung derart eingestellt ist, dass sie am Grund der Vertiefungen (4) grösser ist als im Bereich der Seitenflanken der Erhebungen (3) oder auf den Erhebungen.

9. Verfahren zum Herstellen eines Implantates (1) nach einem der Ansprüche 1 bis 8, umfassend die Schritte
- Bereitstellen eines unbeschichteten Implantatgrundkörpers (8), wobei der Implantatgrundkörper mindestens teilweise eine Oberflächenstruktur (2) aufweist, welche Erhebungen (3) und Vertiefungen (4) umfasst,
- Aufbringen einer porösen Beschichtung (5) im Bereich mit der Oberflächenstruktur derart,
- dass sich die poröse Beschichtung der Kontur der Vertiefungen (4) folgend erstreckt.

10. Verfahren nach Anspruch 9, **dadurch gekennzeichnet, dass** das Aufbringen der porösen Beschichtung (5) derart selektiv erfolgt, dass beschichtete Zonen (6) im Bereich der Vertiefungen (4) an unbeschichtete Zonen (7a) im Bereich der Erhebungen (3) angrenzen.

11. Verfahren nach Anspruch 10, **dadurch gekennzeichnet, dass** das selektive Aufbringen der porösen Beschichtung (5) durch Auftragen einer Schicht im Bereich der Erhebungen (3) erfolgt, die ein Anhaften der porösen Beschichtung (5) weitestgehend verhindert.

12. Verfahren nach Anspruch 9, **dadurch gekennzeichnet, dass** die poröse Beschichtung (5) über den gesamten Bereich mit der Oberflächenstruktur (2) aufgebracht wird und dass anschliessend die poröse Beschichtung im Bereich der Erhebungen (3) derart abgetragen wird, dass die Schichtdicke der porösen Beschichtung auf den Erhebungen kleiner ist als in den Vertiefungen.

13. Verfahren nach Anspruch 12, **dadurch gekennzeichnet, dass** das Abtragen der porösen Beschichtung (5) im Bereich der Erhebungen (3) durch Kugelstrahlen, Abscheren, Bürsten, Drehen, Fräsen, Schleifen, Gleitschleifen (Trowalisieren), chemisches oder elektrochemisches Abtragen erfolgt.

14. Verfahren nach einem der Ansprüche 9 bis 13, **dadurch gekennzeichnet, dass** das Aufbringen der porösen Beschichtung (5) durch ein Plasmabeschichten, insbesondere durch ein atmosphärisches Plasmaspritzverfahren (APS) oder ein Vakuum Plasmaspritzverfahren (VPS), durch Präzipitation, oder Additive Manufacturing erfolgt.

15. Verfahren nach einem der Ansprüche 9 bis 14, **dadurch gekennzeichnet, dass** das Verfahren zusätzlich den Schritt
- Sintern des mit der porösen Beschichtung (5) beschichteten Grundkörpers (8) umfasst.

## Claims

1. Implant (1), in particular a hip-joint socket implant, for implantation into a bone, wherein the implant (1) has, at least in parts, a surface structure (2) comprising elevations (3) and depressions (4), wherein the depressions (4) have zones (6) with a porous coating (5), wherein
a) the elevations (3) have uncoated zones (7a) which adjoin the coated zones (6) in the depressions,
b) or the elevations (3) have coated zones (7b) which adjoin the coated zones (6) in the depressions, wherein the layer thickness of the porous coating (5) is smaller on the elevations than in the depressions (4),
c) or the elevations have coated zones (7b) which adjoin the coated zones (6) in the depressions, wherein the coating is less porous on the elevations than in the depressions,
d) or the elevations (3) have coated zones (7b) which adjoin the coated zones (6) in the depressions, wherein the abrasion resistance of the porous coating (5) is greater on the elevations than in the depressions (4),
and wherein the porous coating (5) extends in a manner following the contour of the depressions (4) .

2. Implant (1) according to Claim 1, **characterized in that** the layer thickness (d) of the porous coating (5) in the depressions (4) amounts to between 15% and 50% of the profile depth (t).

3. Implant (1) according to either of Claims 1 and 2, **characterized in that** the layer thickness (d) of the porous coating (5) in the depressions (4) is between 0.2 mm and 0.5 mm.

4. Implant (1) according to one of Claims 1 to 3, **characterized in that** the implant (1) comprises a main body (8) consisting of titanium, zirconium, niobium, tantalum, or alloys thereof, cobalt-chromium alloy, medical steel, ceramic or polyethylene.

5. Implant (1) according to one of Claims 1 to 4, **characterized in that** the implant (1) has a porous coating (5) consisting of titanium, zirconium, niobium, tantalum, or alloys thereof, cobalt-chromium alloy.

6. Implant (1) according to one of Claims 1 to 5, **characterized in that** the porous coating (5) is a particle coating and preferably has particles (9) which are spherical, cubic or cylindrical in shape.

7. Implant (1) according to Claim 6, **characterized in that** the porous coating (5) has particles (9) with an average size of 200 µm to 500 µm.

8. Implant (1) according to one of Claims 1 to 7, **characterized in that** the porosity of the coating is set in such a manner that it is greater at the base of the depressions (4) than in the region of the side flanks of the elevations (3) or on the elevations.

9. Method for producing an implant (1) according to one of Claims 1 to 8, the method comprising the steps of
- providing an uncoated implant main body (8), wherein the implant main body has, at least in parts, a surface structure (2) comprising elevations (3) and depressions (4),
- applying a porous coating (5) in the region with the surface structure (2) in such a manner
- that the porous coating extends in a manner following the contour of the depressions (4).

10. Method according to Claim 9, **characterized in that**
- the porous coating (5) is applied selectively in such a manner that coated zones (6) in the region of the depressions (4) adjoin uncoated zones (7a) in the region of the elevations (3).

11. Method according to Claim 10, **characterized in that** the porous coating (5) is selectively applied by applying a layer in the region of the elevations (3) which prevents adhesion of the porous coating (5) to the greatest possible extent.

12. Method according to Claim 9, **characterized in that** the porous coating (5) is applied over the entire region with the surface structure (2) and **in that** the porous coating (5) is subsequently removed in the region of the elevations (3), in such a manner that the layer thickness of the porous coating is smaller on the elevations than in the depressions.

13. Method according to Claim 12, **characterized in that** the porous coating (5) is removed in the region of the elevations (3) by shot blasting, shearing, brushing, turning, milling, grinding, barrel finishing, chemical or electrochemical removal.

14. Method according to one of Claims 9 to 13, **characterized in that** the porous coating (5) is applied by plasma coating, in particular by an atmospheric plasma spraying method (APS) or a vacuum plasma spraying method (VPS), by precipitation, or additive manufacturing.

15. Method according to one of Claims 9 to 14, **characterized in that** the method additionally comprises the step of
- sintering the main body (8) coated with the porous coating (5).

## Revendications

1. Implant (1), en particulier implant de la cavité de l'articulation de la hanche, destiné à être implanté dans un os, au moins certaines parties de l'implant (1) présentant une structure de surface (2) qui comporte des saillies (3) et des creux (4), les creux (4) présentant des zones (6) dotées d'un revêtement poreux (5),
a) les saillies (3) présentant des zones (7a) non revêtues adjacentes aux zones revêtues (6) dans les creux,
b) les saillies (3) présentent des zones revêtues (7b) adjacentes aux zones revêtues (6) dans les creux, l'épaisseur du revêtement poreux (5) étant plus petite sur les saillies que dans les creux (4),
c) les saillies présentent des zones revêtues (7b) adjacentes aux zones revêtues (6) dans les creux, le revêtement étant moins poreux sur les saillies que dans les creux, ou
d) les saillies (3) présentent des zones revêtues (7b) adjacentes aux zones revêtues (6) dans les creux, la résistance à l'abrasion du revêtement poreux (5) étant plus grande sur les saillies que dans les creux (4),
**caractérisé en ce que**
le revêtement poreux (5) s'étend en suivant le contour des creux (4).

2. Implant (1) selon la revendication 1, **caractérisé en ce que** l'épaisseur (d) du revêtement poreux (5) dans les creux (4) représente entre 15 % et 50 % de la profondeur (t) du profil.

3. Implant (1) selon l'une des revendications 1 ou 2, **caractérisé en ce que** l'épaisseur (d) du revêtement poreux (5) dans les creux (4) est comprise entre 0,2 mm et 0,5 mm.

4. Implant (1) selon l'une des revendications 1 à 3, **caractérisé en ce que** l'implant (1) comporte un corps de base (8) constitué de titane, de zirconium, de niobium, de tantale ou de leurs alliages, d'un alliage de cobalt et de chrome, d'un acier médical, de céramique ou de polyéthylène.

5. Implant (1) selon l'une des revendications 1 à 4, **caractérisé en ce que** l'implant (1) présente un revêtement poreux (5) constitué de titane, de zirconium, de niobium, de tantale ou de leurs alliages, ou d'un alliage de cobalt et de chrome.

6. Implant (1) selon l'une des revendications 1 à 5, **caractérisé en ce que** le revêtement poreux (5) est un revêtement en particules et présente des particules (9) de préférence de forme sphérique, cubique ou cylindrique.

7. Implant (1) selon la revendication 6, **caractérisé en ce que** le revêtement poreux (5) présente des particules (9) dont la taille moyenne est comprise entre 200 µm et 500 µm.

8. Implant (1) selon l'une des revendications 1 à 7, **caractérisé en ce que** la porosité du revêtement est ajustée de telle sorte qu'elle soit plus grande au fond des creux (4) qu'au niveau des flancs latéraux des saillies (3) ou sur les saillies.

9. Procédé de fabrication d'un implant (1) selon l'une des revendications 1 à 8, le procédé comportant les étapes qui consistent à :
préparer un corps de base (8) d'implant non revêtu, au moins certaines parties du corps de base de l'implant présentant une structure de surface (2) qui comporte des saillies (3) et des creux (4),
appliquer un revêtement poreux (5) sur les parties présentant une structure de surface, de telle sorte que le revêtement poreux s'étende en suivant le contour des creux (4).

10. Procédé selon la revendication 9, **caractérisé en ce que** l'application du revêtement poreux (5) s'effectue de manière sélective de telle sorte que des zones revêtues (6) au niveau des creux (4) soient adjacentes à des zones non revêtues (7a) au niveau des saillies (3).

11. Procédé selon la revendication 10, **caractérisé en ce que** l'application sélective du revêtement poreux (5) s'effectue par application d'une couche au niveau des saillies (3), cette couche empêchant largement une adhérence sur le revêtement poreux (5).

12. Procédé selon la revendication 9, **caractérisé en ce que** le revêtement poreux est appliqué sur toute la zone dotée de la structure de surface (2) et **en ce qu'**ensuite le revêtement poreux est enlevé au niveau des saillies (3) de telle sorte que l'épaisseur du revêtement poreux soit plus petite sur les saillies que dans les creux.

13. Procédé selon la revendication 12, **caractérisé en ce que** l'enlèvement du revêtement poreux (5) au niveau des saillies (3) s'effectue par projection de billes, arrachage, brossage, tournage, fraisage, meulage, polissage (polissage dit "de Trowal") ou enlèvement chimique ou électrochimique.

14. Procédé selon l'une des revendications 9 à 13, **caractérisé en ce que** l'application du revêtement poreux (5) s'effectue par revêtement au plasma, en particulier par procédé de projection atmosphérique d'un plasma (APS) ou un procédé de projection de plasma sous vide (VPS), par précipitation ou par fabrication additive.

15. Procédé selon l'une des revendications 9 à 14, **caractérisé en ce que** le procédé comporte en outre l'étape de frittage du corps de base (8) revêtu par le revêtement poreux (5).
